# EUROPEAN PATENT APPLICATION

(11) **EP 4 721 656 A1**
(43) Date of publication of application: **08.04.2026**
(21) Application number: 24815330.6
(22) Date of filing: 22.05.2024
(51) Int. Cl.: A61B 5/0245, A61B 5/0255, G06F 3/01

(54) **PULSATION INFORMATION TRANSMISSION DEVICE AND CARDIAC-PULSATION INFORMATION TRANSMISSION PROGRAM**

(30) Priority: 29.05.2023 JP 2023088095; 21.03.2024 JP 2024044882
(71) Applicant: JVCKenwood Corporation, Yokohama-shi, Kanagawa 2210022 (JP)
(72) Inventor: SUGAWARA, Takashi, Yokohama-shi, Kanagawa 221-0022 (JP)
(74) Representative: Wagner & Geyer
(86) International application number: PCT/JP2024/018841
(87) International publication number: WO 2024/247846

(57) **Abstract**

A heartbeat information transmission device (2A to 2E) includes a processor (212A to 212E), and a memory (212A to 212E) including instructions that, when executed by the processor (212A to 212E), cause the processor (212A to 212E) to perform the following processing. The processing includes: acquiring a heartbeat signal (HBS) based on a heartbeat of a heart; generating a plurality of delay signals (DLS) repeated for each delay time (DT) while attenuating the acquired heartbeat signal (HBS) at an attenuation rate; and generating a heartbeat output signal (HBLS) for controlling at least one of a light emission or a vibration, the heartbeat output signal (HBLS) being obtained by adding the generated plurality of delay signals (DLS) to the acquired heartbeat signal (HBS).

## Description

### [TECHNICAL FIELD]

The present disclosure relates to a heartbeat information transmission device and a heartbeat information transmission program.

### [BACKGROUND ART]

Patent Literature 1 describes a communication device that acoustically outputs synthesized speech and simultaneously emits an optical signal depending on the semantic content of the synthesized speech, to visually support understanding of the speech.

On the other hand, in recent years, communication using various types of biological information, such as heartbeat, tactile sense, and body temperature, has been studied. Focusing on transmission of the heartbeat, a technique of visually expressing and transmitting detection signals, such as a sound generated by heartbeat (heartbeat sound), an electromotive force (electrocardiogram) generated by the heartbeat, and a pulse wave, as flickering of light has been studied.

### [CITATION LIST]

### [PATENT LITERATURE]

[Patent Literature 1] JP 2009-500679 A

### [SUMMARY OF INVENTION]

In a case where a detection signals, such as a heartbeat sound, an electrocardiogram, and a pulse wave, are directly converted into optical signals or vibration signals, flickering of light or vibration becomes instantaneous pulses, and thus it is difficult to express and transmit feeling of heart beating.

An object of the present disclosure is to provide a heartbeat information transmission device and a heartbeat information transmission program that are capable of expressing and transmitting feeling of heart beating by as flickering of light or vibration, using a heartbeat signal based on the heartbeat.

A heartbeat information transmission device in accordance with some embodiments includes a processor, and a memory including instructions that, when executed by the processor, cause the processor to perform the following processing. The processing includes: acquiring a heartbeat signal based on a heartbeat of a heart; generating a plurality of delay signals repeated for each delay time while attenuating the acquired heartbeat signal at an attenuation rate; and generating a heartbeat output signal for controlling at least one of a light emission or a vibration, the heartbeat output signal being obtained by adding the generated plurality of delay signals to the acquired heartbeat signal.

A heartbeat information transmission program in accordance with some embodiments causes a computer to execute: acquiring a heartbeat signal based on a heartbeat of a heart; generating a plurality of delay signals repeated for each delay time while attenuating the acquired heartbeat signal at an attenuation rate; and generating a heartbeat output signal for controlling at least one of a light emission or a vibration, the heartbeat output signal being obtained by adding the generated plurality of delay signals to the acquired heartbeat signal.

According to the above configuration, the feeling of heart beating can be expressed and transmitted as flickering of light or vibration, using the heartbeat signal based on the heartbeat.

### [BRIEF DESCRIPTION OF DRAWINGS]

[FIG. 1] FIG. 1 is a block diagram illustrating a configuration of a heartbeat information transmission system including a heartbeat information transmission device and a heartbeat detection device according to a first embodiment.
[FIG. 2] FIG. 2 is a functional block diagram of the microcontroller in FIG. 1.
[FIG. 3] FIG.3 is a graph illustrating an example of a heartbeat light signal.
[FIG. 4A] FIG. 4A is a graph illustrating a heartbeat light signal in a case where an attenuation rate is set to a value at which a delay signal is sufficiently attenuated during a heartbeat cycle.
[FIG. 4B] FIG. 4B is a graph illustrating a heartbeat light signal in a case where the attenuation rate is set to a value larger than a value at which the delay signal is sufficiently attenuated during the heartbeat cycle.
[FIG. 5] FIG. 5 is a graph illustrating an example of temporal changes of a heartbeat signal and a heartbeat light signal generated using the heartbeat signal.
[FIG. 6] FIG.6 is a functional block diagram of a microcontroller according to a second embodiment.
[FIG. 7] FIG. 7 is a block diagram illustrating a configuration of a heartbeat information transmission system including a heartbeat information transmission device and a heartbeat detection device according to a third embodiment.
[FIG. 8] FIG. 8 is a functional block diagram of the microcontroller in FIG. 7.
[FIG. 9] FIG. 9 is a flowchart illustrating an example of a heartbeat information transmission method executed by a microcontroller according to a heartbeat information transmission program.
[FIG. 10] FIG. 10 is a block diagram illustrating a configuration of a heartbeat information transmission system including a heartbeat information transmission device and a heartbeat detection device according to a fifth embodiment.
[FIG. 11] FIG. 11 is a block diagram illustrating a configuration of a heartbeat information transmission system including a heartbeat information transmission device and a heartbeat detection device according to a sixth embodiment.
[FIG. 12] FIG. 12 is a block diagram illustrating a configuration of a heartbeat information transmission system including a heartbeat information transmission device, a heartbeat detection device, and an output device according to a seventh embodiment.
[FIG. 13] FIG. 13 is a block diagram illustrating a configuration of the output device according to the seventh embodiment.
[FIG. 14] FIG. 14 is an external view of the output device according to the seventh embodiment.
[FIG. 15] FIG. 15 is an external view of the output device according to the seventh embodiment.

### [DESCRIPTION OF EMBODIMENTS]

Hereinafter, a plurality of embodiments will be described with reference to the accompanying drawings. In the description of the drawings described below, the same or similar parts are denoted by the same or similar reference numerals.

The following embodiments exemplify an apparatus and a method for embodying a technical idea, and does not specify the material, shape, structure, arrangement, and the like of each component. The embodiments can be modified in various ways in the claims.

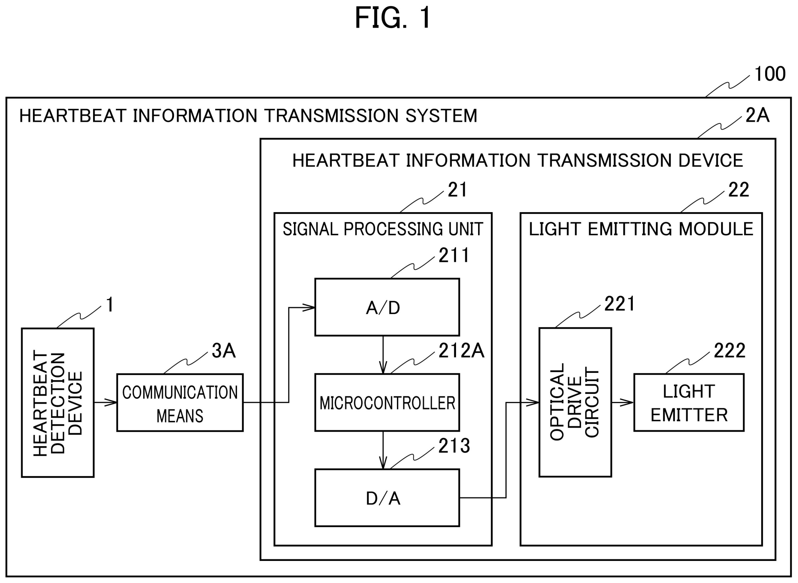

A configuration of a heartbeat information transmission system 100 including a heartbeat information transmission device 2A and a heartbeat information transmission device 2A according to a first embodiment will be described with reference to FIG. 1. The heartbeat information transmission system 100 includes the heartbeat detection device 1 that detects a heartbeat signal HBS based on a heartbeat, and the heartbeat information transmission device 2A that expresses and transmits feeling of heart beating by flickering of light, using the heartbeat signal HBS detected by the heartbeat detection device 1.

The "heartbeat" refers to a motion in which the heart cyclically repeats contraction and relaxation. Blood is pumped out by the heartbeat. The number of times the heartbeats per unit time is referred to as a heart rate. The "heartbeat signal HBS based on the heartbeat" includes electrical signals obtained by detecting a heartbeat sound that is a sound generated by the heartbeat, a weak electromotive force generated by the heartbeat, a pulse that is a beat of an artery, and the like. Further, the "heartbeat signal HBS" includes an electrocardiographic signal from an electrocardiographic sensor attached to a chest and a pulse wave signal obtained by measuring a change in blood flow (hemoglobin) with an optical sensor attached to a wrist or the like. The heartbeat is also called "cardiac beat". The heartbeat sound is also called a sound of a heart beating. A graph of the temporal change of the weak electromotive force corresponds to the electrocardiogram.

The heartbeat detection device 1 includes all devices that detect the heartbeat signal HBS including electric signals such as heartbeat sound, a weak electromotive force, and a pulse, and examples of the devices include an electrocardiographic sensor attached to a chest and an optical pulse wave sensor attached to a wrist or the like. For example, the heartbeat detection device 1 may include a stethoscope that detects vibration in an audible range generated from the heart or an artery, and a microphone that converts the detected vibration into an electric signal. The microphone may directly detect the vibration in the audible range without using the stethoscope.

The heartbeat signal HBS detected by the heartbeat detection device 1 is transmitted to the heartbeat information transmission device 2A via the communication means 3A. The communication means 3A includes all communication facilities and communication equipment for transmitting the heartbeat signal HBS to the heartbeat information transmission device 2A by wire or wirelessly. The communication means 3A connects the heartbeat detection device 1 and the heartbeat information transmission device 2A via at least one of a cable, Bluetooth (registered trademark), Wi-Fi (registered trademark), or the Internet. The communication means 3A may be a computer network which is a communication facility for transmitting information by connecting communication terminals, such as a personal computer or a smartphone, and various servers by a wired or wireless connection. According to the communication means 3A, the heartbeat detection device 1 and the heartbeat information transmission device 2A can be configured as individual devices.

The heartbeat information transmission device 2A includes a signal processing unit 21 and a light emitting module 22.

The signal processing unit 21 processes the heartbeat signal HBS received from the heartbeat detection device 1 via the communication means 3A, to generate and output a heartbeat light signal HBLS as a heartbeat output signal that expresses and transmits the feeling of heart beating by flickering of light. For example, the signal processing unit 21 includes an A/D conversion circuit 211, a microcontroller 212A, and a D/A conversion circuit 213. The A/D conversion circuit 211 converts the heartbeat signal HBS (analog signal) received via the communication means 3A into a digital signal. The microcontroller 212A processes the heartbeat signal HBS, which is a digital signal, to generate the heartbeat light signal HBLS. The D/A conversion circuit 213 converts the heartbeat light signal HBLS, which is a digital signal, into an analog signal. The signal processing unit 21 outputs a heartbeat light signal HBLS which is an analog signal. The A/D conversion circuit 211, the microcontroller 212A, and the D/A conversion circuit 213 can be implemented with, for example, electronic components mounted on a printed circuit board. The A/D conversion circuit 211 and the D/A conversion circuit 213 can be implemented using a known circuit technology. Note that the A/D conversion circuit 211 can be omitted in terms of functionality in a case where the heartbeat signal HBS is digitally transmitted by the communication means 3A.

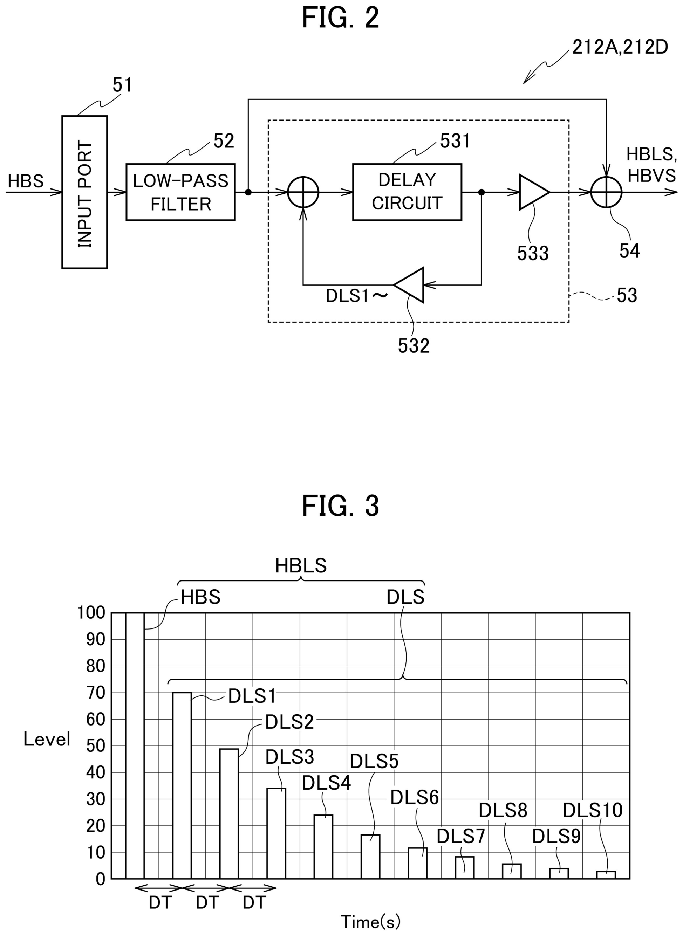

The microcontroller 212A is a general-purpose microcontroller including a central processing unit (CPU) which is a processor, a memory, and an input/output port. A computer program (heartbeat information transmission program) to be described later is installed and executed in the microcontroller 212A. As a result, the microcontroller 212A functions as a plurality of information processing means 53 and 54 to be described later with reference to FIG. 2. Here, although an example in which a part of the heartbeat information transmission device 2A is implemented by software is described, it is, needless to say, also possible to prepare dedicated hardware for executing each information processing and configure the plurality of information processing means 53 and 54. The dedicated hardware includes devices such as application specific integrated circuits (ASIC), e.g., a digital signal processor (DSP), and conventional circuit components.

The light emitting module 22 includes a light emitter 222 that emits light based on the heartbeat light signal HBLS output from the signal processing unit 21, and an optical drive circuit 221 that drives the light emitter 222. The configuration of the light emitter 222 is not particularly limited as long as the luminance can be changed according to the intensity of the heartbeat light signal HBLS. For example, the light emitter 222 may have a configuration in which a known light emitter, such as an incandescent lamp, a light emitting diode (LED), or a vertical cavity surface emitting laser (VCSEL), is combined with a diffusion lens or the like. The circuit configuration of the optical drive circuit 221 is not limited as long as it can change the luminance of the light emitted from the light emitter 222 depending on the intensity of the heartbeat light signal HBLS.

FIG. 2 is a functional block diagram of the microcontroller 212A. As illustrated in FIG. 2, the microcontroller 212A includes an input port 51 which is an example of a heartbeat signal acquisition unit, a low-pass filter 52, a delay signal generator 53, and an adder circuit 54. The delay signal generator 53 and the adder circuit 54 are examples of information processing means that can be implemented by the above-described heartbeat information transmission program.

As illustrated in FIGS. 2 and 3, the microcontroller 212A acquires the heartbeat signal HBS from the input port 51. The microcontroller 212A outputs the acquired heartbeat signal HBS to the delay signal generator 53, and outputs the acquired heartbeat signal HBS to the adder circuit 54 while bypassing the delay signal generator 53. The delay signal generator 53 generates a plurality of delay signals DLS1, DLS2, DLS3, DLS4, DLS5, DLS6, DLS7, DLS8, DLS9, and DLS10 repeated at intervals of the delay time DT while attenuating the input heartbeat signal HBS at an attenuation rate. Hereinafter, the plurality of delay signals DLS1 to DLS10 are collectively referred to as a plurality of delay signals DLS. FIG. 3 illustrates 10 delay signals DLS1 to DLS10, but the number of the plurality of delay signals DLS is not limited to 10. The adder circuit 54 generates and outputs the heartbeat light signal HBLS obtained by adding the plurality of delay signals DLS output from the delay signal generator 53 to the heartbeat signal HBS. The light emitting module 22 in FIG. 1 emits light based on the heartbeat light signal HBLS output from the adder circuit 54. The adder circuit 54 adds the plurality of delay signals DLS to the heartbeat signal HBS acquired by the input port 51 and bypassing the delay signal generator 53, to generate and output the heartbeat light signal HBLS. The microcontroller 212A outputs a heartbeat light signal HBLS which is a digital signal.

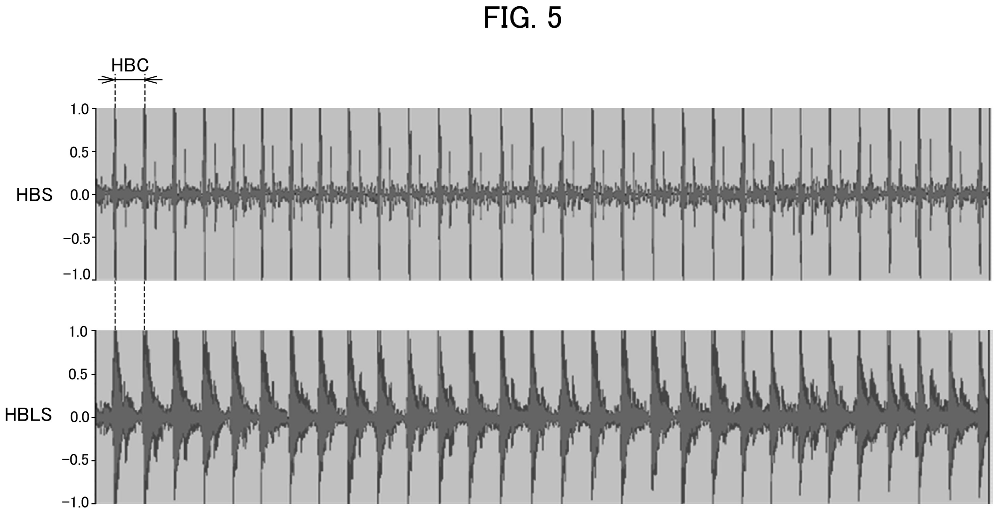

FIG. 5 is a graph illustrating an example of temporal changes of a heartbeat signal HBS and a heartbeat light signal HBLS generated using the heartbeat signal HBS. The vertical axis represents the signal intensity of the heartbeat signal HBS and the heartbeat light signal HBLS, and the horizontal axis represents time. As illustrated in FIG. 5, the heartbeat signal HBS is a signal whose intensity changes in a discontinuous or pulsed manner in a cycle (heartbeat cycle) HBC in which the heart beats. In a case where the heartbeat signal HBS is directly converted into an optical signal, flickering of light is discontinuous or pulsed, and thus, it is not possible to express and transmit the feeling of heart beating. Therefore, the signal processing unit 21 generates a heartbeat light signal HBLS obtained by adding a plurality of delay signals DLS to the heartbeat signal HBS. By sufficiently shortening the delay time DT in FIG. 3, the heartbeat light signal HBLS obtained by adding the plurality of delay signals DLS to the heartbeat signal HBS can be visually recognized as one continuous optical signal that attenuates at a prescribed attenuation rate. The light emitting module 22 is caused to emit light based on the heartbeat light signal HBLS. Due to the afterimage effect of human beings, the light emission of the light emitting module 22 is visually recognized as one continuous light that attenuates at a prescribed attenuation rate, and the feeling of heart beating can be expressed and transmitted by flickering of light.

Returning to FIG. 2, the delay signal generator 53 includes a delay circuit 531, an attenuator 532, and a second attenuator 533. The delay circuit 531 generates a delay signal obtained by delaying the input heartbeat signal HBS by the delay time DT. The delay circuit 531 also outputs the generated delay signal to the attenuator 532 and the second attenuator 533. The attenuator 532 attenuates the delay signal output from the delay circuit 531 at an attenuation rate. The delay signals DLS1 to DLS9 attenuated by the attenuator 532 are input again to the delay circuit 531. The second attenuator 533 attenuates the intensity of the plurality of delay signals DLS output from the delay circuit 531 before inputting the signals to the adder circuit 54. Each of the delay time DT and the attenuation rate is a prescribed value. As a result, it is possible to generate a plurality of delay signals DLS repeated at intervals of the delay time DT while attenuating the heartbeat signal HBS at the attenuation rate.

The low-pass filter 52 is for removing noise included in the heartbeat signal HBS acquired from the input port 51, and removes a noise component at a prescribed frequency or more from the heartbeat signal HBS. The heartbeat signal HBS output from the low-pass filter 52 is input to the delay signal generator 53 and the adder circuit 54. The microcontroller 212A may not include the low-pass filter 52.

With the second attenuator 533, it is possible to collectively reduce an entire intensity of the plurality of delay signals DLS. In the example illustrated in FIG. 2, although the delay signal generator 53 includes the delay circuit 531, the attenuator 532, and the second attenuator 533, the circuit configuration is not limited thereto. The delay signal generator 53 only needs to have a circuit configuration capable of generating a plurality of delay signals repeated at intervals of the delay time DT while attenuating the input heartbeat signal HBS at an attenuation rate.

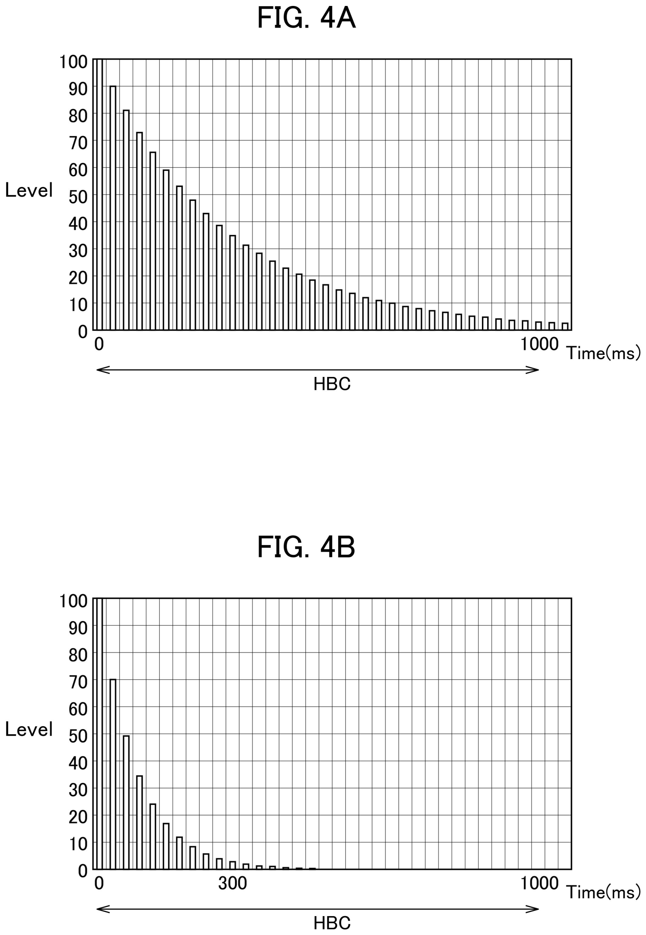

FIGS. 4A and 4B are graphs illustrating two heartbeat light signal HBLS having different attenuation rates. The vertical axis represents the intensity of the heartbeat light signal HBLS, and the horizontal axis represents time. In the first embodiment, as illustrated in FIG. 4A, the attenuation rate is set to a value at which the delay signal DLS is sufficiently attenuated during the heartbeat cycle HBC. Thus, the feeling of heart beating can be expressed as the flickering of light. If the attenuation rate is too small, the delay signal DLS is not sufficiently attenuated at the time of the next heartbeat, so that the change in the luminance of light becomes smaller and the feeling of heart beating cannot be expressed. On the other hand, if the attenuation rate is too large, as illustrated in FIG. 4B, the delay signal DLS attenuates to almost zero before the time of the next heartbeat, and thus the flickering of light is discontinuous or pulsed, and the feeling of heart beating cannot be expressed.

For example, as illustrated in FIG. 4A, the attenuation rate may be set to a value at which the delay signal DLS attenuates to several% at the time of the next heartbeat. Thus, the feeling of heart beating can be expressed as the flickering of light.

The delay time DT is set to be equal to or less than an afterimage duration that can be recognized as continuous lighting due to an afterimage effect of human beings. Alternatively, the delay time DT may be set to be equal to or less than the time resolution of human vision. Hereinafter, the time resolution of human vision and the afterimage duration are collectively noted as an afterimage duration. As a result, since the plurality of delay signals DLS repeated at intervals of the delay time is visually recognized as one continuous optical signal, the feeling of heart beating can be expressed as flickering of light.

For example, the delay time DT is 100 ms or less. Although the afterimage duration varies depending on the intensity, color, and the like of light, it is said that the time during which human beings continuously perceive an afterimage on average is 100 sm. Therefore, when the delay time DT is 100 ms or less, the plurality of delay signals DLS repeated in every delay time DT is visually recognized as one continuous optical signal. On the other hand, if the delay time DT is too short, the delay signal DLS is attenuated before the time of the next heartbeat, and the feeling of heart beating cannot be expressed. Therefore, a lower limit value of the delay time DT is set to a value at which the delay signal attenuates to several % at the time of the next heartbeat.

FIG. 4A illustrates a heartbeat light signal HBLS in a case where the delay time DT is 30 ms, a gain (amplification factor) is 0.9, the heart rate is 60 beats per minute, and the heartbeat cycle HBC is 1000 ms. In FIG. 4A, the delay signal DLS is generated 33 times in total during the heartbeat cycle HBC, and the delay signal DLS is sufficiently attenuated (to about 3%) at the time of the next heartbeat.

On the other hand, FIG. 4B illustrates the heartbeat light signal HBLS in a case where the gain (amplification factor) is set to 0.7 and the other conditions are the same as those in FIG. 4A. In FIG. 4B, since the attenuation rate is too high, that is, the gain is too low, the delay signal DLS is already attenuated to about 3% after 300 ms from the heartbeat. In this case, the blinking of the light is discontinuous or pulsed like the cardiac beat signal.

In the first embodiment, the signal processing unit 21 may be provided in the heartbeat detection device 1. Alternatively, some functions in the signal processing unit 21, for example, the A/D conversion circuit 211 and the low-pass filter 52 may be provided in the heartbeat detection device 1. Alternatively, the heartbeat detection device 1 and the heartbeat information transmission device 2A may be configured as the same device. By appropriately selecting and using any of these configurations, the configuration in the system can be simplified according to the embodiment.

### (Second Embodiment)

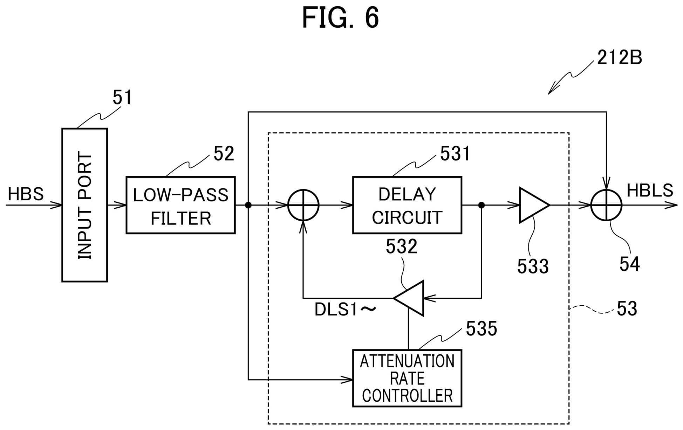

A configuration of a microcontroller 212B according to a second embodiment will be described with reference to FIG. 6. The microcontroller 212B is different from the microcontroller 212A illustrated in FIG. 2 in that it further includes an attenuation rate controller 535 that controls an attenuation rate on the basis of a heart rate, but is identical in other points. The configuration of the heartbeat information transmission device 2A other than the microcontroller 212A and the heartbeat information transmission system 100 are the same as those of the first embodiment.

The attenuation rate controller 535 can calculate a heartbeat cycle HBC from the heart rate and control the attenuation rate so that a delay signal is sufficiently attenuated during the heartbeat cycle HBC. Specifically, the attenuation rate controller 535 increases the attenuation rate as the heart rate increases (as the heartbeat cycle HBC decreases). As a result, even if the heartbeat cycle HBC changes, the attenuation rate can be controlled so that the delay signal is sufficiently attenuated during the heartbeat cycle HBC. Therefore, feeling of heart beating can be expressed as flickering of light regardless of the level of the heart rate.

The heartbeat signal HBS is input to the attenuation rate controller 535, and the heartbeat cycle HBC is calculated. The attenuation rate controller 535 has a data table indicating a correspondence relationship between the heartbeat cycle HBC and the attenuation rate, or a calculation function of obtaining the attenuation rate using the heartbeat cycle HBC as a variable in advance. The attenuation rate controller 535 can calculate the attenuation rate at which the delay signal is sufficiently attenuated during the heartbeat cycle HBC, using the data table or the calculation function.

The attenuation rate controller 535 controls the attenuation rate so that the delay signal DLS is attenuated during the heartbeat cycle HBC of the heart. Thus, the feeling of heart beating can be expressed as the flickering of light. If the attenuation rate is too small, the delay signal is not sufficiently attenuated at the time of the next heartbeat, so that the change in the luminance of light becomes smaller and the feeling of heart beating cannot be expressed. On the other hand, if the attenuation rate is too large, as illustrated in FIG. 4B, the delay signal DLS attenuates to almost zero before the time of the next heartbeat, and thus the flickering of light is discontinuous or pulsed, and the feeling of heart beating cannot be expressed.

The attenuation rate controller 535 controls the attenuation rate so that the delay signal attenuates to several% at the time of the next heartbeat of the heart. Thus, the feeling of heart beating can be expressed as the flickering of light.

The attenuation rate controller 535 may perform control of maintaining one of the attenuation rate and the delay time DT constant and adjusting the other, so that the delay signal DLS is attenuated during the heartbeat cycle HBC of the heart. Alternatively, the attenuation rate controller 535 may perform control to simultaneously adjust both the attenuation rate and the delay time DT. The attenuation rate controller 535 may perform control of adjusting the delay time DT instead of or in addition to the attenuation rate, so that the delay signal DLS is attenuated during the heartbeat cycle HBC of the heart. That is, the attenuation rate controller 535 may perform control to adjust at least one of the attenuation rate or the delay time, so that the delay signal DLS is attenuated during the heartbeat cycle of the heart.

The attenuation rate controller 535 described above may continuously perform control operation or may perform the control operation in every prescribed period on the basis of the average value of the heartbeat cycles HBC within the prescribed period.

### (Third Embodiment)

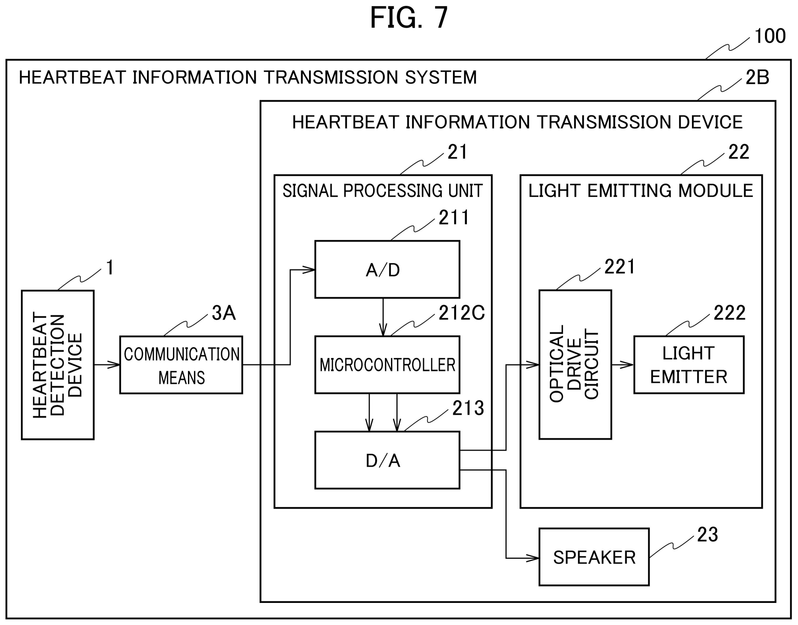

A configuration of a heartbeat information transmission system 100 including a heartbeat information transmission device 2B and a heartbeat detection device 1 according to a third embodiment will be described with reference to FIG. 7. The heartbeat information transmission system 100 of FIG. 7 is identical to the heartbeat information transmission system 100 of FIG. 1 except that the heartbeat information transmission device 2B further includes a speaker 23 and that the configuration of a microcontroller 212C is different from the configuration of the microcontroller 212A of FIG. 2. The speaker 23 is an example of an output unit configured to output vibration from a heartbeat signal HBS. The speaker 23 includes a diaphragm, vibrates the diaphragm in an audible range, and transmits the vibration to air or the like.

The speaker 23 is configured to output a sound from the heartbeat signal HBS. The speaker 23 receives the heartbeat signal HBS (analog signal) output from a D/A conversion circuit 213 of a signal processing unit 21. The speaker 23 outputs a sound based on the heartbeat signal HBS. Any type of speaker 23 such as a voice coil speaker or a MEMS speaker may be used regardless of a driving system of the speaker 23 and a type of the diaphragm. The D/A conversion circuit 213 has two channels, and simultaneously outputs the heartbeat signal HBS and a heartbeat light signal HBLS. Although the heartbeat signal HBS is a pulse-like signal, the sound output from the speaker 23 can express and transmit the feeling of heart beating. In a case where the heartbeat detection device 1 is a microphone and directly detects the vibration in the audible range, the heartbeat information transmission device 2B may directly output the heartbeat signal HBS acquired via a communication means 3A as an audio signal to the speaker 23 without the signal processing unit 21.

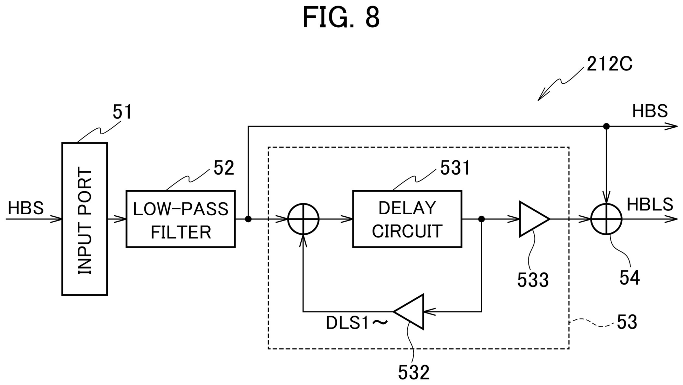

FIG. 8 is a functional block diagram of the microcontroller 212C of FIG. 7. The microcontroller 212C is different from the microcontroller 212A of FIG. 2 in that the microcontroller 212C outputs the heartbeat signal HBS output from a low-pass filter 52, but is identical in other points. The output heartbeat signal HBS (digital signal) is input to the D/A conversion circuit 213 in FIG. 7, converted into an analog signal, and then input to the speaker 23.

According to the third embodiment, it is possible to simultaneously output an optical signal and an audio signal from the heartbeat signal HBS based on heartbeat. Note that the third embodiment may be implemented in combination with the second embodiment including the attenuation rate controller 535. Furthermore, the heartbeat information transmission device 2B may include a vibrator that vibrates the diaphragm instead of the speaker 23 or in addition to the speaker 23. The vibrator is another example of the output unit configured to output vibration from the heartbeat signal HBS. The vibrator outputs vibration of the diaphragm in accordance with the heartbeat. By bringing a part of the human body of a user into contact with the diaphragm, such as by placing the user's hand on the diaphragm included in the vibrator, the feeling of heart beating can be expressed and transmitted by the vibration of the diaphragm. The feeling of heart beating can be expressed and transmitted not only by the sound which is vibration of the air, but also by the vibration of the diaphragm itself.

### (Fourth Embodiment)

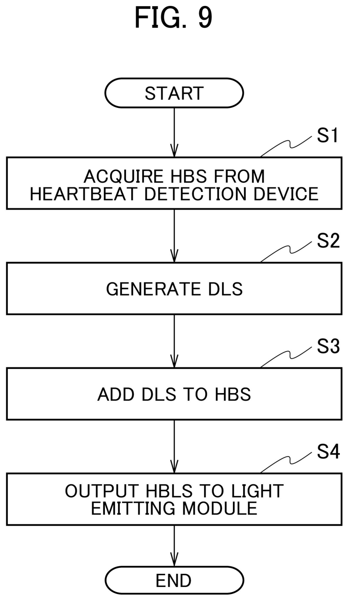

In the fourth embodiment, an example of a computer program (heartbeat information transmission program) installed and executed in a microcontroller 212A will be described with reference to FIG. 9. FIG. 9 is a flowchart illustrating an example of a heartbeat information transmission method executed by the microcontroller 212A according to the heartbeat information transmission program.

The heartbeat information transmission program is a computer program that causes microcontrollers 212A and 212C, which are examples of computers, to function as an input port 51 which is an example of a heartbeat signal acquisition unit, a delay signal generator 53, and an adder circuit 54. As illustrated in FIG. 9, first, in step S1, the input port 51 acquires a heartbeat signal HBS based on the heartbeat. The process proceeds to step S2, and the delay signal generator 53 generates a plurality of delay signals DLS repeated at intervals of delay time DT while attenuating the heartbeat signal HBS at an attenuation rate. In step S3, the adder circuit 54 generates a heartbeat light signal HBLS obtained by adding the plurality of delay signals DLS output from the delay signal generator 53, to the heartbeat signal HBS acquired by the input port 51 and bypassing the delay signal generator 53. Finally, in step S4, the microcontroller 212A outputs the heartbeat light signal HBLS from the output port to a light emitting module 22.

By sufficiently shortening the delay time DT, the heartbeat light signal HBLS obtained by adding the plurality of delay signals DLS to the heartbeat signal HBS can be visually recognized as one continuous optical signal that attenuates at a prescribed attenuation rate. Therefore, according to the heartbeat information transmission program, it is possible to generate the heartbeat light signal HBLS for expressing and transmitting the feeling of heart beating by flickering of light, using the heartbeat signal HBS based on the heartbeat.

### (Fifth Embodiment)

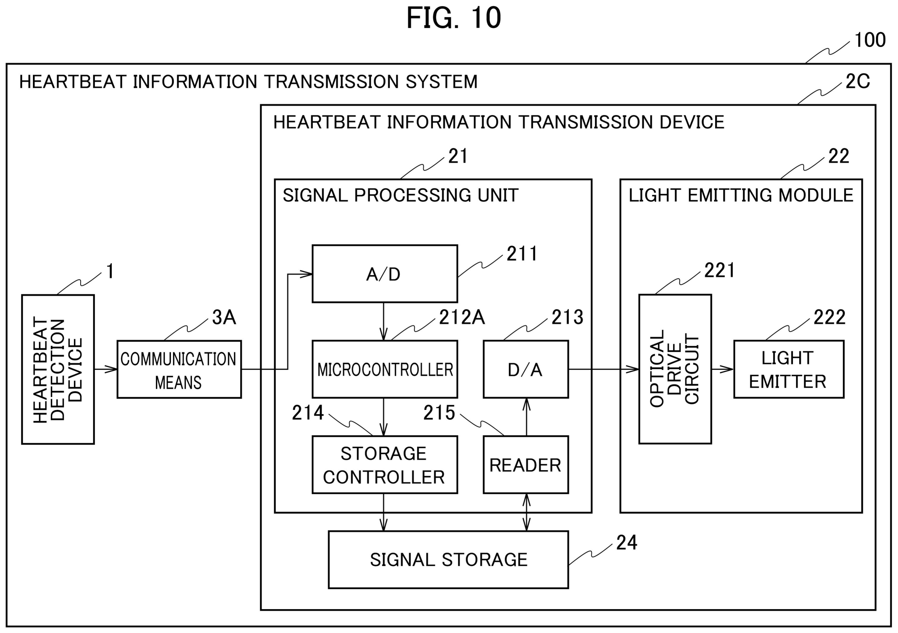

A configuration of a heartbeat information transmission system 100 including a heartbeat information transmission device 2C and a heartbeat detection device 1 according to a fifth embodiment will be described with reference to FIG. 10. The heartbeat information transmission system 100 of FIG. 10 is identical to the heartbeat information transmission system 100 of FIG. 1 except that the heartbeat information transmission device 2C further includes a signal storage 24 and that a signal processing unit 21 includes a storage controller 214 and a reader 215. The storage controller 214 causes the signal storage 24 to store a heartbeat light signal HBLS generated by a microcontroller 212A. The reader 215 reads the heartbeat light signal HBLS stored in the signal storage 24 at any timing, for example, timing specified by a user, and transmits the heartbeat light signal HBLS to a D/A conversion circuit 213.

According to the fifth embodiment, by providing the signal storage 24 that stores the heartbeat light signal HBLS generated by the microcontroller 212A, a light emitting module 22 can be caused to emit light based on the heartbeat light signal HBLS generated from heartbeat, at any timing different from the timing at which the heartbeat detection device 1 detects the heartbeat.

In the present embodiment, the signal stored in the signal storage 24 is not limited to the heartbeat light signal HBLS, and may be a heartbeat signal HBS acquired from an input port 51 or a heartbeat signal HBS output from a low-pass filter 52. The signal processing unit 21 may read the stored heartbeat signal HBS at any timing, for example, timing specified by the user, and generate and output the heartbeat light signal HBLS. In a case where the heartbeat light signal HBLS is stored in the signal storage 24, as compared with a case where the heartbeat signal HBS is stored, it is not necessary to perform the process of generating the heartbeat light signal HBLS when the stored signal is read because it is specified by the user or the like, and the process at the time of reading can be simplified.

### (Sixth Embodiment)

In the third embodiment, the example in which the signal processing unit 21 vibrates the diaphragm by the heartbeat signal HBS has been described. Meanwhile, in a sixth embodiment, a case in which a vibration module is vibrated by a heartbeat vibration signal HBVS to which delay signal addition processing has been applied similarly to the heartbeat light signal HBLS will be described.

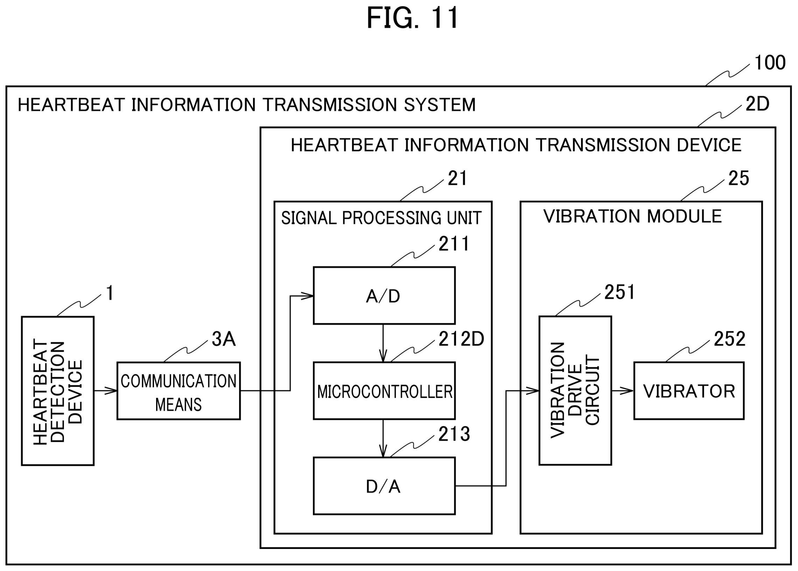

A configuration of a heartbeat information transmission system 100 including a heartbeat information transmission device 2D and a heartbeat detection device 1 according to the sixth embodiment will be described with reference to FIG. 11. The heartbeat information transmission system 100 of FIG. 11 is different from the heartbeat information transmission device 2A illustrated in FIG. 1 in that a microcontroller 212D in a signal processing unit 21 processes a heartbeat signal HBS, which is a digital signal, to generate the heartbeat vibration signal HBVS as a heartbeat output signal and that a module communicably connected to a signal processing unit 21 is a vibration module 25, but is identical in other points.

As illustrated in FIG. 2, the microcontroller 212D generates a plurality of delay signals DLS similarly to the microcontroller 212A, and generates the heartbeat vibration signal HBVS obtained by adding the generated delay signals DLS to the heartbeat signal HBS.

The vibration module 25 includes a vibrator 252 that vibrates on the basis of the heartbeat vibration signal HBVS output from the signal processing unit 21, and a vibration drive circuit 251 that drives the vibrator 252. The vibrator 252 can be configured using, for example, a haptic device that vibrates a weight by electromagnetic drive, a piezoelectric drive type device, an electrostatic drive type device, or the like. For example, the vibrator 252 may be configured using a haptic device in consideration of a balance between vibration and energy. The vibrator 252 may have any circuit configuration as long as an amplitude of the vibration module 25 can be adjusted so as to vibrate at the intensity indicated by the heartbeat vibration signal HBVS.

According to the sixth embodiment, feeling of heart beating can be expressed and transmitted as a vibration, by using the heartbeat vibration signal HBVS output from the heartbeat signal HBS based on the heartbeat.

### (Seventh Embodiment)

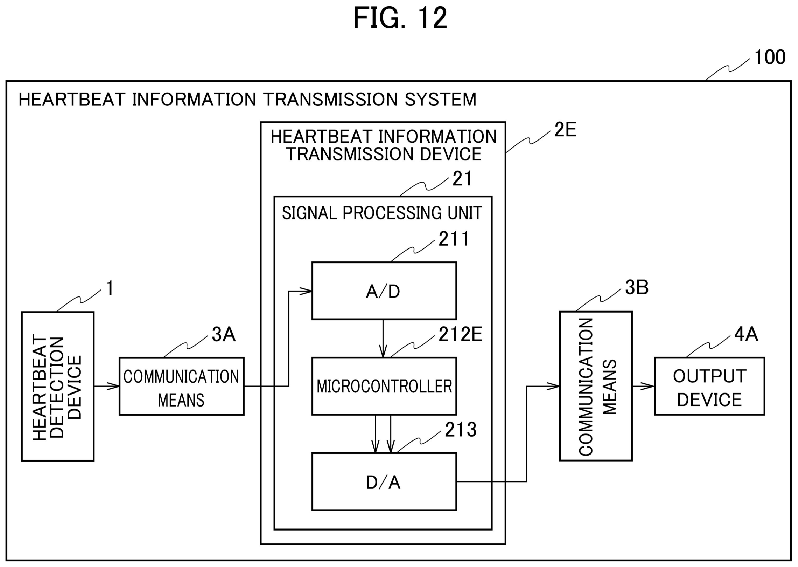

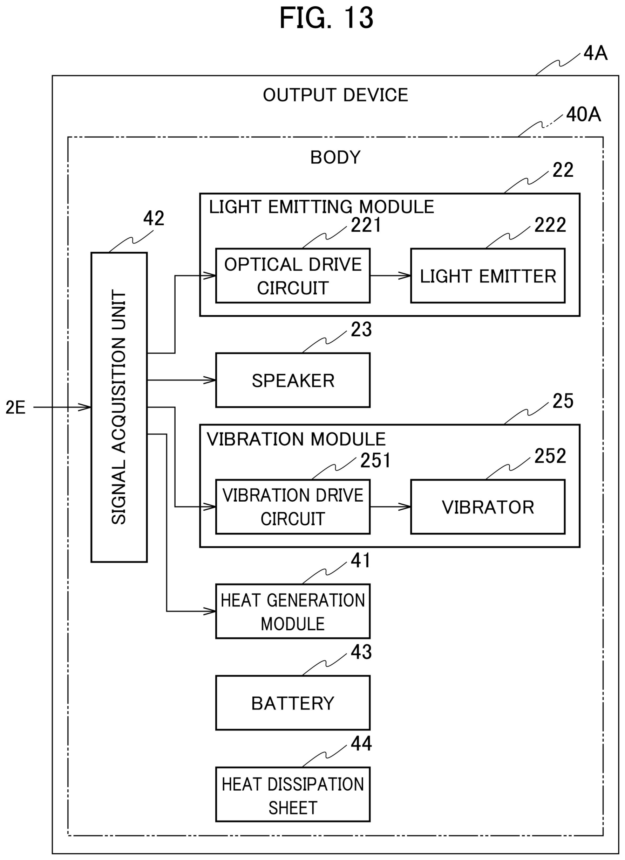

A configuration of a heartbeat information transmission system 100 including a heartbeat information transmission device 2E, a heartbeat detection device 1, and an output device 4A according to a seventh embodiment will be described with reference to FIGS. 12 and 13. The heartbeat information transmission system 100 of FIG. 12 is different from the heartbeat information transmission device 2A illustrated in FIG. 1 in that an output module, such as a light emitting module 22, is provided in an output device 4A which is a medium different from the heartbeat information transmission device 2E.

The heartbeat information transmission device 2E according to the present embodiment includes a signal processing unit 21. The microcontroller 212E in the signal processing unit 21 processes a heartbeat signal HBS, which is a digital signal, to generate a heartbeat light signal HBLS and a heartbeat vibration signal HBVS, as a heartbeat output signal. The microcontroller 212E may generate one type of signal shared as the heartbeat light signal HBLS and the heartbeat vibration signal HBVS, or may generate different signals as the HBLS and HBVS. The signal generated by the signal processing unit 21 is transmitted to the output device 4A via a communication means 3A.

As illustrated in FIG. 13, the output device 4A includes a light emitting module 22, a speaker 23, a vibration module 25, and a heat generation module 41 as output modules in a body 40A having a prescribed shape, and further includes a signal acquisitor 42, a battery 43, and a heat dissipation sheet 44. The signal acquisition unit 42 acquires a signal transmitted from the heartbeat information transmission device 2E.

A light emitter 222 of the light emitting module 22 may include two light emitting diodes (LEDs), and an optical drive circuit 221 may connect the respective polarities of these two LEDs in reverse parallel to a positive pole and a negative pole of the battery 43 to emit light. By configuring the light emitter 222 with two LEDs in this manner, light can be emitted with higher brightness.

The heat generation module 41 is configured using, for example, a Peltier element. When the heartbeat light signal HBLS or the heartbeat vibration signal is acquired from the signal processing unit 21, heat is generated such that the surface of the body 40A has a temperature equivalent to the body temperature of a general human body. In addition, the heat generation module 41 may generate heat such that the surface of the body 40A is at a temperature equivalent to the body temperature of a person whose heartbeat is detected that is measured by a thermometer (not illustrated). The battery 43 supplies power to the light emitting module 22, the speaker 23, the vibration module 25, the heat generation module 41, and the signal acquisition unit 42. The heat dissipation sheet 44 improves the thermal conductivity in the body 40A and efficiently conducts heat generated by the heat generation module 41 to the surface of the body 40A.

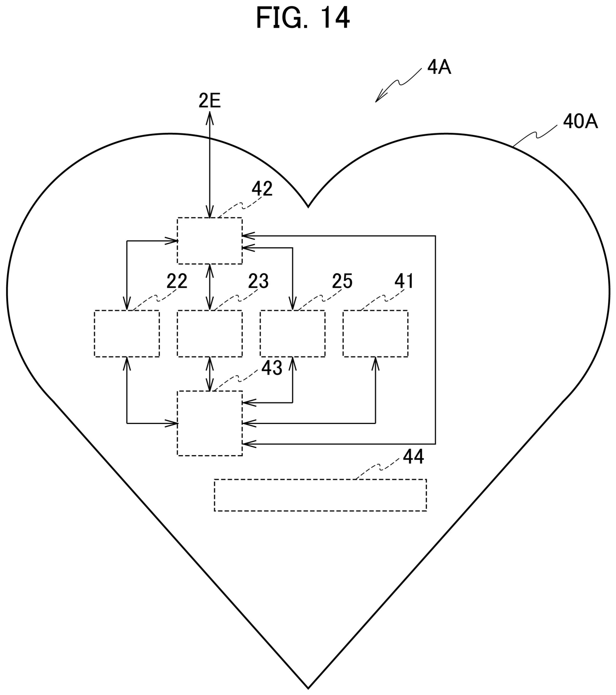

FIG. 14 is an external view of the output device 4A. The output device 4A is a portable device, and the body 40A is formed in a heart shape with a size that can be accommodated in a palm of a person using resin. A speaker 23, a vibration module 25, a heat generation module 41, a signal acquisition unit 42, a battery 43, and a heat dissipation sheet 44 are provided in the body 40A in addition to the light emitting module 22.

In the output device 4A, the light emitting module 22 emits light, the speaker 23 outputs a sound, the vibration module 25 vibrates, and the heat generation module 41 generates heat, on the basis of a signal transmitted from the heartbeat information transmission device 2E.

According to the seventh embodiment, from the heartbeat signal HBS based on the heartbeat, the optical signal, the vibration signal, and the audio signal expressing feeling of heart beating can be simultaneously output from the portable output device 4A. In addition, in the output device 4A, when a signal expressing the feeling of heart beating is output, the surface of the body 40A is set to a temperature equivalent to the body temperature of the human body, and the feeling of heart beating can be more realistically recognized by a user who holds the output device 4A at a place away from the beating information transmission device 2E.

In the present embodiment, the output device 4A may be provided with at least one of the light emitting module 22, the speaker 23, the vibration module 25, or the heat generation module 41.

In the present embodiment, the heartbeat detection device 1 may be configured by a microphone to acquire the heartbeat signal HBS, or a microphone may be further provided separately from the heartbeat detection device 1 configured by a sensor or the like. In the case of using the microphone in this manner, the collected sound may be output from the speaker 23 via the heartbeat information transmission device 2E. At this time, the heartbeat information transmission device 2E may not provide a low-pass filter for a signal to be output as a sound, or may set a cutoff frequency of the low-pass filter to be higher than that in the case of processing the heartbeat signal HBS. With this setting, the sound can be output with high accuracy.

As described above, the heartbeat signal HBS acquired using the microphone may be branched, one signal may be used to generate the heartbeat light signal HBLS by removing a noise component with a low-pass filter, and the other signal may output the heartbeat signal HBS as a sound from the speaker 23 without passing through the low-pass filter.

The bone conduction speaker may be installed by integrating the speaker 23 and the vibration module 25 in the output device 4A. By configuring the heartbeat information transmission system 100 using the bone conduction speakers in this manner, it is possible to cause the user of the output device 4A to more realistically recognize the feeling of heart beating.

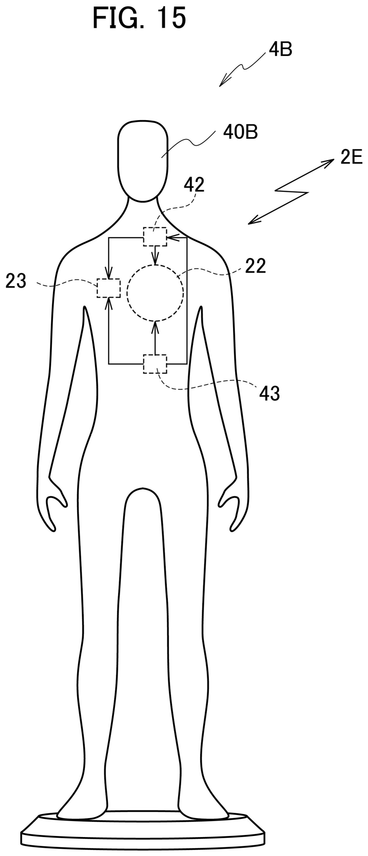

As a device that outputs the heartbeat output signal generated from the heartbeat sound, an output device 4B having a body 40B formed in a human-like appearance as illustrated in FIG. 15 may be used. The output device 4B includes a light emitting module 22 and a speaker 23 as output modules and further includes a signal acquisition unit 42 and a battery 43, and causes the light emitting module 22 to emit light on the basis of the heartbeat output signal and causes the speaker 23 to output a sound.

As a device that outputs the heartbeat output signal generated from the heartbeat sound, an information processing device such as a smart device including a display and a speaker may be used. The information processing device causes the display to display, for example, an avatar image as a light emitting module, causes the avatar image to emit light on the basis of a heartbeat output signal, and causes a speaker to output a sound.

The present invention is not limited to the embodiments described above, and various modifications can be made without departing from the gist of the present invention. At least one of Bluetooth (registered trademark), Wi-Fi (registered trademark), and the Internet may be used to establish a wireless connection between the heartbeat detection device and the heartbeat information transmission device, between the heartbeat information transmission device and the output device, or between the heartbeat detection device and the output device. As a result, the heartbeat of the user wearing the heartbeat detection device can be transmitted to a heartbeat information transmission device or an output device possessed by another user by at least one of flickering of light or vibration.

The heartbeat information transmission devices 2A to 2E can be realized using a general-purpose electronic device including electronic components corresponding to the functions of the respective devices, for example, a smartphone, a tablet PC, or a laptop PC. These electronic devices are wirelessly connected to the heartbeat detection device 1 via the communication means 3A, and the heartbeat information transmission program is installed and executed in the signal processing unit 21 in the electronic device, whereby the heartbeat information transmission devices 2A to 2E and the heartbeat information transmission system 100 can be realized.

The entire contents of Japanese Patent Application No. 2023-088095 (filing date: May 29, 2023) and Japanese Patent Application No. 2024-044882 (filing date: March 21, 2024) are incorporated herein by reference.

## Claims

1. A heartbeat information transmission device comprising:
a processor; and
a memory including instructions that, when executed by the processor, cause the processor to perform the following processing,
wherein the processing includes:
acquiring a heartbeat signal based on a heartbeat of a heart;
generating a plurality of delay signals repeated for each delay time while attenuating the acquired heartbeat signal at an attenuation rate; and
generating a heartbeat output signal for controlling at least one of a light emission or a vibration, the heartbeat output signal being obtained by adding the generated plurality of delay signals to the acquired heartbeat signal.

2. The heartbeat information transmission device according to claim 1, wherein the processing further includes outputting the generated heartbeat output signal to at least one of a light emitting module or a vibration module.

3. The heartbeat information transmission device according to claim 1 or 2, further comprising at least one of a light emitting module configured to emit light based on the heartbeat output signal generated by the processing or a vibration module configured to vibrate based on the heartbeat output signal generated by the processing.

4. The heartbeat information transmission device according to any one of claims 1 to 3, wherein, when generating the plurality of delay signals, the processing includes:
generating a delay signal obtained by delaying the acquired heartbeat signal by the delay time;
attenuating the generated delay signal at an attenuation rate; and
further delaying the attenuated delay signal by the delay time.

5. The heartbeat information transmission device according to any one of claims 1 to 4, wherein the processing further includes controlling the attenuation rate based on a heart rate of the heart.

6. The heartbeat information transmission device according to claim 1, further comprising:
a light emitting module configured to emit light based on the heartbeat output signal generated by the processing; and
an output unit configured to output a sound or a vibration from the heartbeat signal acquired by the processing.

7. The heartbeat information transmission device according to claim 3, further comprising an output unit configured to output a sound from the heartbeat signal acquired by the processing.

8. A heartbeat information transmission program for causing a computer to execute:
acquiring a heartbeat signal based on a heartbeat of a heart;
generating a plurality of delay signals repeated for each delay time while attenuating the acquired heartbeat signal at an attenuation rate; and
generating a heartbeat output signal for controlling at least one of a light emission or a vibration, the heartbeat output signal being obtained by adding the generated plurality of delay signals to the acquired heartbeat signal.
